(19) **Europäisches Patentamt**
**European Patent Office**
**Office européen des brevets**

(11) **EP 3 854 313 B1**

(12) **EUROPEAN PATENT SPECIFICATION**

(45) Date of publication and mention
of the grant of the patent:
**13.09.2023 Bulletin 2023/37**

(21) Application number: **21152799.9**

(22) Date of filing: **21.01.2021**

(51) International Patent Classification (IPC):
*A61B 8/08* *(2006.01)* *G06T 7/00* *(2017.01)*

(52) Cooperative Patent Classification (CPC):
**A61B 8/085; A61B 8/0825; A61B 8/5207;
A61B 8/5223; G06T 7/0016;** G06T 2207/10016;
G06T 2207/10136

(54) **ULTRASOUND DIAGNOSTIC APPARATUS AND METHOD OF CONTROLLING THE SAME**

ULTRASCHALLDIAGNOSEVORRICHTUNG UND VERFAHREN ZUR STEUERUNG DAVON

APPAREIL DE DIAGNOSTIC À ULTRASONS, SON PROCÉDÉ DE COMMANDE ET PRODUIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.01.2020 KR 20200008399**

(43) Date of publication of application:
**28.07.2021 Bulletin 2021/30**

(73) Proprietor: **Samsung Medison Co., Ltd.
Hongcheon-gun, Gangwon-do 25108 (KR)**

(72) Inventors:
• **KIM, Kang Sik
05340 Seoul (KR)**

• **KIM, Jung Ho
05340 Seoul (KR)**
• **YOO, Yang Mo
07214 Seoul (KR)**
• **KANG, Jin Bum
02794 Seoul (KR)**
• **HAN, Kang Hee
06326 Seoul (KR)**

(74) Representative: **Arnold & Siedsma
Bezuidenhoutseweg 57
2594 AC The Hague (NL)**

(56) References cited:
**US-A1- 2012 232 390 US-A1- 2014 364 733**

**EP 3 854 313 B1**

## Description

BACKGROUND

1. Field

The disclosure relates to an ultrasound diagnostic apparatus and a method of controlling the same, and a computer program product, and more particularly, to a technology for generating and displaying an image representing micro-calcification tissues in the ultrasound diagnostic apparatus.

2. Description of Related Art

A production of micro-calcification according to the accumulation of calcium in a human body causes disorders in organs or cardiovascular function in the human body and manifests various diseases. Particularly, micro-calcification tissues present in a breast are known to have a high possibility of metastasis to malignant tumors, and thus a technology capable of detecting the micro-calcification tissues at an early stage is required to diagnose breast cancer early.

In a conventional micro-calcification detection technology, mainly X-ray images can be used in consideration of the fact that calcareous material absorbs relatively little X-rays. However, this technology has disadvantages that it is impossible to detect the micro-calcification tissues in real time, the accessibility of the user is poor, and there is a potential risk (blood flow apoptosis, cancer expression, DNA mutation) due to exposure of a patient to radiation. In addition, in clinically reading the micro-calcification tissues, evaluation of objectivity and standardized criteria are insufficient.

On the other hand, an ultrasound diagnostic apparatus irradiates an ultrasound signal generated from a transducer of a probe to an object, receives information of a signal reflected from the object, and obtains at least one image of a portion (for example, soft tissue or blood flow) inside the object.

The ultrasound diagnostic apparatus is harmless to the human body and has the advantage of being able to observe an internal structure and characteristics of the human body noninvasively. The ultrasound diagnostic apparatus may provide various types of clinical information (tissue shape, elasticity, blood flow rate, etc.). In particular, the ultrasound diagnostic apparatus may be used for monitoring micro-calcification, or for real-time monitoring for biopsy of micro-calcification tissues. In this case, breast ultrasound or musculoskeletal ultrasound may be used.

In recent years, the importance of a method of efficiently detecting a plurality of micro-calcification tissues distributed at different depths or different positions within the object is increasing.

An apparatus according to the preamble of claim 1 and a corresponding method are known from US 2012/232390 A1.

SUMMARY

An aspect of the disclosure provides an ultrasound diagnostic apparatus that detects a plurality of micro-calcification tissues distributed at different depths or positions, and displays the detected micro-calcification tissues as an image.

Additional aspects of the disclosure will be set forth in part in the description which follows and, in part, will be obvious from the description, or may be learned by practice of the disclosure.

According to an aspect of the disclosure, there is provided an ultrasound diagnostic apparatus according to claim 1.

The first position of the object may be a position that is a different depth from the second position and is spaced apart from each other. The controller may be configured to set the first position and the second position of the object as a first focal point and a second focal point to which the transmitted second ultrasound pulse is focused, respectively.

The controller may be configured to control the ultrasound transceiver so that the second ultrasound pulse transmitted to the first position of the object and the second ultrasound pulse transmitted to the second position of the object are transmitted at predetermined time intervals.

The controller may be configured to control the ultrasound transceiver so that the second ultrasound pulse transmitted to the first position of the object and the second ultrasound pulse transmitted to the second position of the object are simultaneously transmitted.

The second ultrasound pulse may be a pulse designed based on characteristics of the micro-calcification tissues.

The second ultrasound pulse may be an asymmetric pulse in which a negative pressure component is dominant compared to a positive pressure component, and have a longer wavelength than the first ultrasound pulse.

In detecting the micro-calcification tissues, the controller may be configured to reconstruct the obtained second and third data into three-dimensional (3D) data including information about an axial depth, a lateral width, and a time, and to extract the micro-calcification tissues by analyzing the 3D data.

In detecting the micro-calcification tissues, the controller may be configured to estimate at least one of an intensity, frequency, or phase of the echo signal reflected from each area of the object by applying singular value decomposition (SVD) to the obtained second and third data, and to detect the micro-calcification tissues in the object based on the estimated value.

In detecting the micro-calcification tissues, the controller is configured to calculate a dispersion of a phase change over time of the echo signal reflected from each area of the object based on the obtained second and third data, and to detect an area in which the dispersion of the phase change is greater than or equal to a predetermined value as the micro-calcification tissues in the object.

The display may be configured to display an index indicating focusing lines in which the set first focal point and second focal point are located in a brightness mode (B-mode) image of a cross section of the object.

The display is configured to display a color bar representing a plurality of colors corresponding to values representing characteristics of the micro-calcification tissues, to select a color from the color bar based on the value representing the characteristics of the detected micro-calcification tissues, and to display the selected color in an area corresponding to the detected micro-calcification tissues included in the ultrasound image.

The value representing the characteristics of the micro-calcification tissues represents a roughness of a phase change over time of the echo signal reflected from the micro-calcification tissues.

The ultrasound image may be a brightness mode (B-mode) image for a cross section of the object. The display may be configured to display an area corresponding to the detected micro-calcification tissues on the ultrasound image.

According to another aspect of the disclosure, there is provided a method of controlling an ultrasound diagnostic apparatus according to claim 11.

The first position of the object may be a position that is a different depth from the second position and is spaced apart from each other. The method may further include setting, by the controller, the first position and the second position of the object as a first focal point and a second focal point to which the transmitted second ultrasound pulse is focused, respectively.

The transmitting of the second ultrasound pulse may further include controlling the second ultrasound pulse transmitted to the first position of the object and the second ultrasound pulse transmitted to the second position of the object to be transmitted at predetermined time intervals.

The transmitting of the second ultrasound pulse may further include controlling the second ultrasound pulse transmitted to the first position of the object and the second ultrasound pulse transmitted to the second position of the object to be simultaneously transmitted.

The detecting of the micro-calcification tissues of the first position and the second position may further include reconstructing the obtained second and third data into three-dimensional (3D) data including information about an axial depth, a lateral width, and a time; and extracting the micro-calcification tissues by analyzing the 3D data.

The detecting of the micro-calcification tissues of the first position and the second position may further include estimating at least one of an intensity, frequency, or phase of the echo signal reflected from each area of the object by applying singular value decomposition (SVD) to the obtained second and third data; and detecting the micro-calcification tissues in the object based on the estimated value.

The detecting of the micro-calcification tissues of the first position and the second position further includes calculating a dispersion of a phase change over time of the echo signal reflected from each area of the object based on the obtained second and third data; and detecting an area in which the dispersion of the phase change is greater than or equal to a predetermined value as the micro-calcification tissues in the object.

The method may further include displaying, by the display, an index indicating focusing lines in which the set first focal point and second focal point are located in a brightness mode (B-mode) image of a cross section of the object.

The displaying of the ultrasound image and the image representing the micro-calcification tissues detected in the first position and the second position further includes displaying, by the display, a color bar representing a plurality of colors corresponding to values representing characteristics of the micro-calcification tissues; selecting, by the display, a color from the color bar based on the value representing the characteristics of the detected micro-calcification tissues; and displaying, by the display, the selected color in an area corresponding to the detected micro-calcification tissues included in the ultrasound image.

The value representing the characteristics of the micro-calcification tissues represents a roughness of a phase change over time of the echo signal reflected from the micro-calcification tissues.

The ultrasound image may be a brightness mode (B-mode) image for a cross section of the object. The displaying of the ultrasound image and the image representing the micro-calcification tissues detected in the first position and the second position may further include displaying an area corresponding to the detected micro-calcification tissues on the ultrasound image.

According to another aspect of the disclosure, there is provided a computer program product including: non-transitory computer readable recording medium storing a computer program code for performing the method of controlling the ultrasound diagnostic apparatus.

BRIEF DESCRIPTION OF THE DRAWINGS

These and/or other aspects of the disclosure will become apparent and more readily appreciated from the following description of the embodiments, taken in conjunction with the accompanying drawings of which:

  FIG. 1 is a block diagram illustrating a configuration of an ultrasound diagnostic apparatus according to an embodiment.
  FIG. 2 are views illustrating an ultrasound diagnostic apparatus according to an embodiment.

FIG. 3 is a view illustrating a structure of an ultrasound diagnostic apparatus capable of independently detecting micro-calcification according to an embodiment.

FIG. 4 is a view illustrating a structure of a controller of an ultrasound diagnostic apparatus according to an embodiment.

FIGS. 5 and 6 are views illustrating setting a focal point at which a second ultrasound pulse is focused according to an embodiment.

FIGS. 7 to 9 are views for describing pulses transmitted to detect micro-calcification according to an embodiment.

FIG. 10 is a view for describing spatiotemporal data generated according to an embodiment.

FIG. 11 is a view for describing characteristics of micro-calcification tissues according to an embodiment.

FIG. 12 is a view illustrating a method of detecting micro-calcification tissues by a micro-calcification detector according to an embodiment.

FIGS. 13 and 14 are views illustrating screens displayed on an ultrasound diagnostic apparatus according to an embodiment.

FIG. 15 is a view illustrating an index representing focusing lines in which a plurality of focal points for a second ultrasound pulse are located, according to an embodiment.

FIG. 16 is a flowchart of a method of displaying an ultrasound image according to an embodiment.

DETAILED DESCRIPTION

Configurations shown in the embodiments and drawings described herein are only embodiments of the disclosed invention, there may be various modifications that can replace the embodiments and drawings of the present specification at the time of filing of the present application.

Like reference numerals refer to like elements throughout the specification. Not all elements of the embodiments of the disclosure will be described, and the description of what are commonly known in the art or what overlap each other in the exemplary embodiments will be omitted. The terms as used throughout the specification, such as "~ part," "~ module," "~ member," "~ block," etc., may be implemented in software and/or hardware, and a plurality of "~ parts," "~ modules," "~ members," or "~ blocks" may be implemented in a single element, or a single "~ part," "~ module," "~ member," or "~ block" may include a plurality of elements.

In the present specification, an "image" may include a medical image obtained by a medical imaging device such as a magnetic resonance imaging (MRI) apparatus, a computed tomography (CT) apparatus, an ultrasound imaging apparatus, or an X-ray imaging apparatus.

In the present specification, an 'object' is to be captured, and may include a person, an animal, or a part thereof. For example, the object may include a portion (e.g., organ) of a human body or a phantom.

Throughout the specification, an "ultrasound image" may refer to an image of the object transmitted to the object and processed based on an ultrasound signal reflected from the object.

Hereinafter, embodiments of the disclosure will be described in detail with reference to the accompanying drawings.

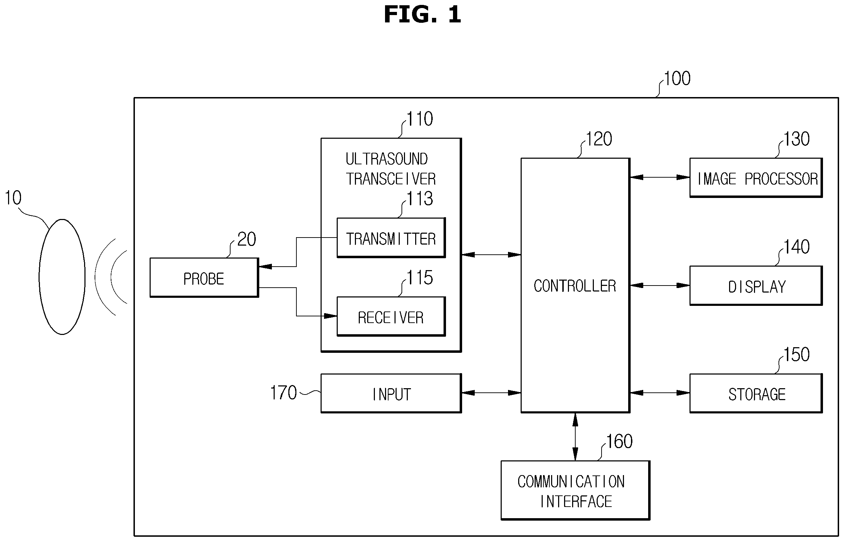

FIG. 1 is a block diagram illustrating a configuration of an ultrasound diagnostic apparatus according to an embodiment.

Referring to FIG. 1, an ultrasound diagnostic apparatus 100 may include a probe 20, an ultrasound transceiver 110, a controller 120, an image processor 130, a display 140, a storage 150, a communication interface 160, and an input 170.

The ultrasound diagnostic apparatus 100 may be implemented not only in a cart type but also in a portable type. Examples of the portable ultrasound diagnostic apparatus may include a smart phone including a probe and an application, a laptop computer, a PDA, and a tablet PC, but are not limited thereto.

The probe 20 may include a plurality of transducers. The plurality of transducers may transmit the ultrasound signal to the object 10 according to a transmission signal applied from a transmitter 113. The plurality of transducers may receive the ultrasound signal reflected from the object 10 and form a received signal. In addition, the probe 20 may be implemented integrally with the ultrasound diagnostic apparatus 100 or may be implemented as a separate type connected to the ultrasound diagnostic apparatus 100 by wire or wireless. In addition, the ultrasound diagnostic apparatus 100 may be provided with one or a plurality of probes 20 depending on an implementation form.

The controller 120 may control the transmitter 113 to form the transmission signal to be applied to each of the plurality of transducers in consideration of positions and focal points of the plurality of transducers included in the probe 20.

The controller 120 may convert the received signal received from the probe 20 to analog to digital, and may control a receiver 115 to generate ultrasound data by summing the digitally converted received signals in consideration of the positions and focal points of the plurality of transducers.

The image processor 130 may generate an ultrasound image by using the ultrasound data generated by the receiver 115.

The display 140 may display the generated ultrasound image and various information processed by the ultrasound diagnostic apparatus 100. The ultrasound diagnostic apparatus 100 may include one or a plurality of displays 140 depending on the implementation form. In addition, the display 140 may be implemented as a touch screen in combination with a touch panel.

The controller 120 may control an overall operation of the ultrasound diagnostic apparatus 100 and signal flow between internal components of the ultrasound diagnostic apparatus 100. The controller 120 may include a memory storing a program or data for performing a function of the ultrasound diagnostic apparatus 100, a processor processing the program or data. In addition, the controller 120 may control the operation of the ultrasound diagnostic apparatus 100 by receiving a control signal from the input 170 or an external device.

The ultrasound diagnostic apparatus 100 may include a communication interface 160, and may be connected to the external device (for example, a server, a medical apparatus, a portable apparatus (smart phone, tablet PC, wearable device, etc.)) through the communication interface 160.

The communication interface 160 may include one or more components enabling communication with the external device, and may include, for example, at least one of a short-range communication module, a wired communication module, and a wireless communication module.

The communication interface 160 may transmit and receive control signals and data with the external devices.

The storage 150 may store various data or programs for driving and controlling the ultrasound diagnostic apparatus 100, input/output ultrasound data, and the obtained ultrasound images.

The input 170 may receive a user input for controlling the ultrasound diagnostic apparatus 100. For example, the user input may include an input for manipulating buttons, a keypad, a mouse, trackballs, jog switches, knobs, etc., an input of touching a touch pad or a touch screen, a voice input, a motion input, a biometric information input (for example, iris recognition, fingerprint recognition, etc.), but the disclosure is not limited thereto.

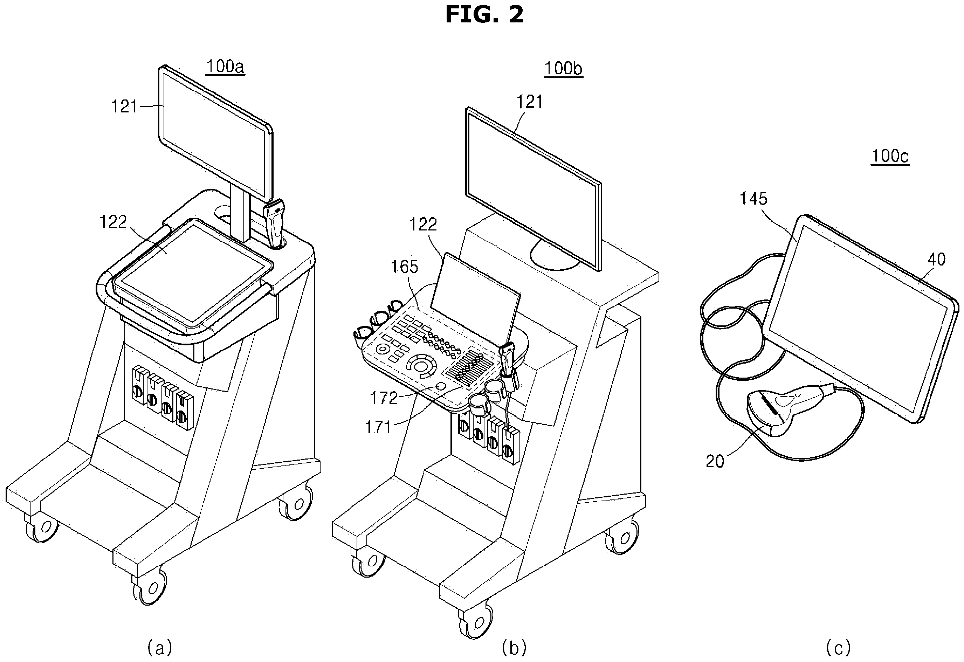

An example of the ultrasound diagnostic apparatus 100 according to an embodiment will be described later through FIGS. 2A to 2C.

FIG. 2 are views illustrating an ultrasound diagnostic apparatus according to an embodiment.

Referring to FIGS. 2A and 2B, the ultrasound diagnostic apparatuses 100a and 100b may include a main display 121 and a sub display 122. One of the main display 121 and the sub display 122 may be implemented as a touch screen. The main display 121 and the sub display 122 may display various types of information processed by the ultrasound diagnostic apparatuses 100a and 100b. In addition, the main display 121 and the sub display 122 are implemented as the touch screen, and may receive data for controlling the ultrasound diagnostic apparatuses 100a and 100b from a user by providing a GUI. For example, the main display 121 may display the ultrasound image, and the sub display 122 may display a control panel for controlling the display of the ultrasound image in a GUI form. The sub display 122 may receive data for controlling the display of an image through the control panel displayed in the GUI form. The ultrasound diagnostic apparatuses 100a and 100b may control the display of the ultrasound image displayed on the main display 121 using the input control data.

Referring to FIG. 2B, the ultrasound diagnostic apparatus 100b may further include a control panel 165 in addition to the main display 121 and the sub display 122. The control panel 165 may include buttons, trackballs, jog switches, knobs, and the like, and may receive data for controlling the ultrasound diagnostic apparatus 100b from the user. For example, the control panel 165 may include a Time Gain Compensation (TGC) button 171, a Freeze button 172, and the like. The TGC button 171 is a button for setting a TGC value for each depth of the ultrasound image. In addition, when the input of the Freeze button 172 is detected while scanning the ultrasound image, the ultrasound diagnostic apparatus 100b may maintain a state in which a frame image at a time point is displayed.

Meanwhile, the buttons, the trackballs, the jog switches, and the knobs included in the control panel 165 may be provided on the main display 121 or the sub display 122 as the GUI.

Referring to FIG. 2C, the ultrasound diagnostic apparatus 100c may also be implemented in a portable type. Examples of the portable ultrasound diagnostic apparatus 100c may include the smart phone including the probe and the application, the laptop computer, the PDA, and the tablet PC, but are not limited thereto.

The ultrasound diagnostic apparatus 100c may include a probe 20 and a body 40, and the probe 20 may be connected to one side of the body 40 by wire or wirelessly. The body 40 may include a touch screen 145. The touch screen 145 may display the ultrasound image, various information processed by the ultrasound diagnostic apparatus 100c, and a GUI.

On the other hand, the ultrasound diagnostic apparatus 100c may provide various types of clinical information including micro-calcification (shape of tissue, elasticity, speed of blood flow, etc.).

The ultrasound diagnostic apparatus 100c may use a method of imaging micro-calcification using a post-processing algorithm of images such as imaging micro-calcification using various beamforming techniques based on channel data, or performing feature enhancement of only hyperechoic components.

However, when the ultrasound diagnostic apparatus 100c uses a method of extracting an area suspected of micro-calcification through post-processing of the ultrasound image, there may be a problem in that it is difficult to identify the presence of calcification tissues and that only micro-calcification tissues cannot be independently detected when information of various human tissues (muscles, fats, blood flow, etc.) is mixed in the area of interest.

When the user identifies the presence of calcification tissue in the object through images with enhanced features of hyperechoic components and performs a diagnosis, depending on the user who reads the ultrasound image, there may be difficulties in standardizing the standard of diagnosis due to low diagnosis agreement. Therefore, there is a need for a technology capable of automatically detecting micro-calcification quickly and accurately, and in particular, development of a technology capable of independently detecting micro-calcification using the ultrasound diagnostic apparatus 100 capable of obtaining real-time images is required.

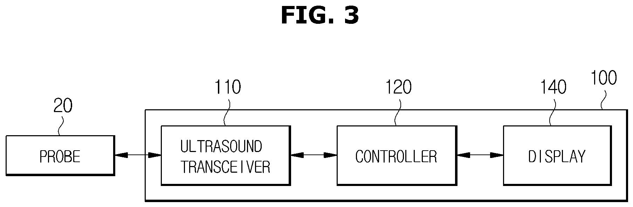

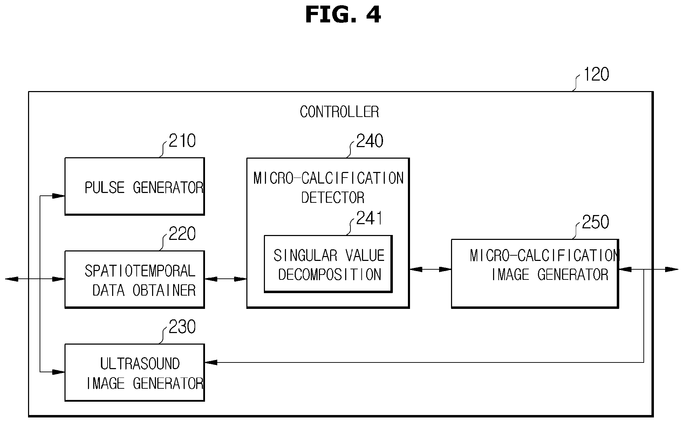

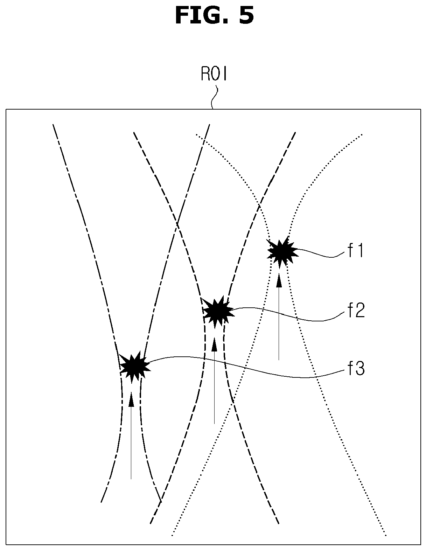

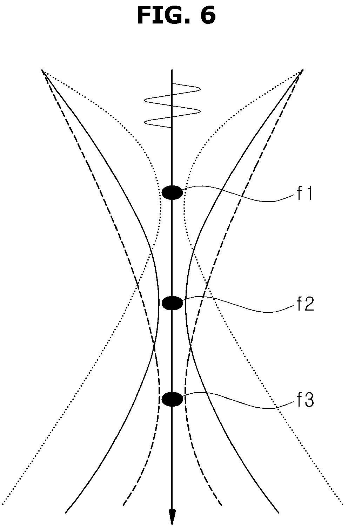

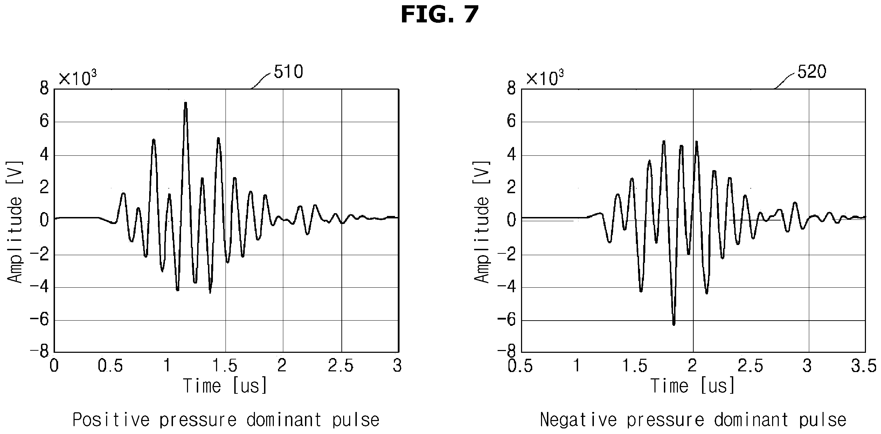

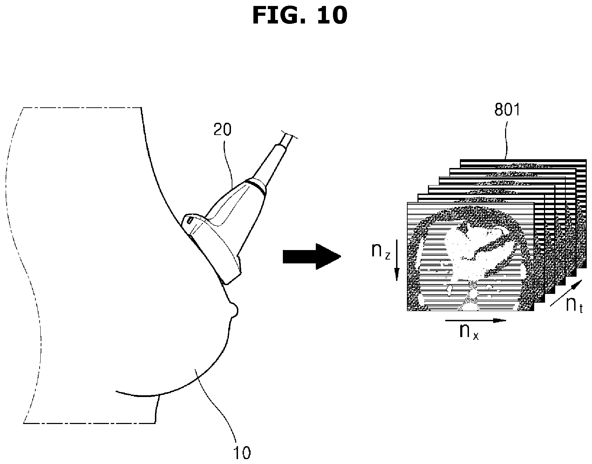

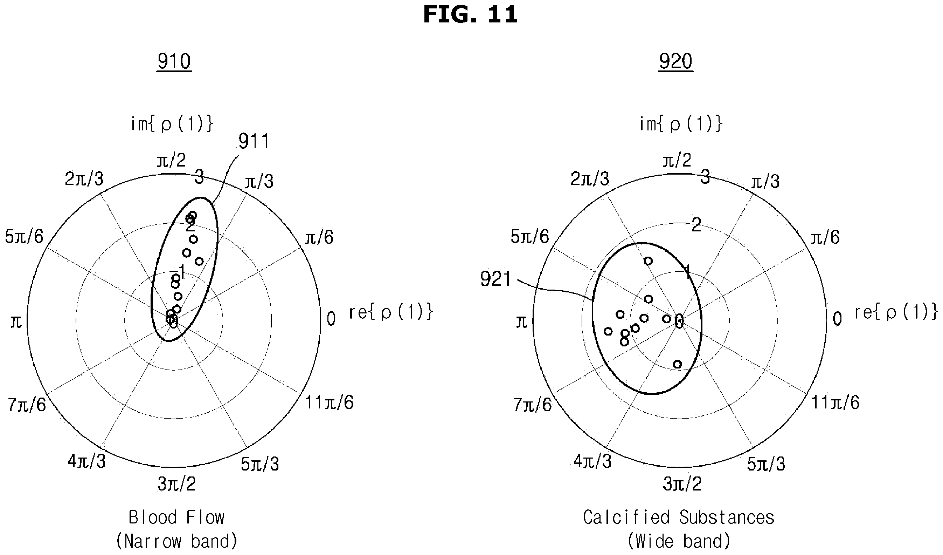

FIG. 3 is a view illustrating a structure of an ultrasound diagnostic apparatus capable of independently detecting micro-calcification according to an embodiment, FIG. 4 is a view illustrating a structure of a controller of an ultrasound diagnostic apparatus according to an embodiment, FIGS. 5 and 6 are views illustrating setting a focal point at which a second ultrasound pulse is focused according to an embodiment, FIGS. 7 to 9 are views for describing pulses transmitted to detect micro-calcification according to an embodiment, FIG. 10 is a view for describing spatiotemporal data generated according to an embodiment, and FIG. 11 is a view for describing characteristics of micro-calcification tissues according to an embodiment.

The ultrasound diagnostic apparatus 100 may include the probe 20, the ultrasound transceiver 110, the controller 120, and the display 140. Each configuration of the ultrasound diagnostic apparatus 100 of FIG. 3 may be applied to the description of the ultrasound diagnostic apparatus 100 of FIG. 1. Therefore, redundant descriptions are omitted.

The ultrasound transceiver 110 may transmit an ultrasound pulse to the object through the probe 20 based on the control signal applied from the controller 120, receive a radio frequency (RF) signal reflected from the object through the probe 20, and output it to the controller 120.

The ultrasound transceiver 110 may obtain first data by allowing the probe 20 to transmit a first ultrasound pulse to the object and receive an echo signal reflected from the object. The first ultrasound pulse may be a pulse designed to obtain a brightness mode (B-mode).

The ultrasound transceiver 110 may obtain second data by allowing the probe 20 to transmit a second ultrasound pulse different from the first ultrasound pulse to the object and repeat the operation of receiving the echo signal reflected from the object a plurality of times at predetermined time intervals.

At this time, the ultrasound transceiver 110 may transmit the second ultrasound pulse through the probe 20 to a plurality of positions such as a first position and a second position of the object, respectively, and receive echo signals reflected from the plurality of positions of the object.

That is, as will be described later, the micro-calcification tissues present in the object may be distributed at various depths or positions. The controller 120 may control the ultrasound transceiver 110 to transmit the second ultrasound pulses simultaneously or at a predetermined time interval to the plurality of positions of the object in which the micro-calcification tissues are located.

The first position and the second position of the object to which the second ultrasound pulse is reached are positions where the micro-calcification tissues are present, and are positions of the focal point preset so that the second ultrasound pulse is reached.

Referring to FIGS. 5 and 6, the controller 120 may set the first position to a third position in which the micro-calcification tissues are present in the object as a first focal point f1 to a third focal point f3 to which the second ultrasound pulse is focused.

The positions of the micro-calcification tissues of the object may have different depths from each other and may be spaced apart from each other, and the number of positions may be plural, so there is no limit to the number of focal points to which the second ultrasound pulse is focused.

As disclosed in FIGS. 5 and 6, the controller 120 may set the first position in which the micro-calcification of the object exists as the first focal point f1 to which the second ultrasound pulse is focused. In addition, the controller 120 may set the second position having a different depth from the first position and having the micro-calcification of a position spaced apart from each other as the second focal point f2 to which the second ultrasound pulse is focused. In addition, the controller 120 may set the third position having a different depth from the first position and the second position and having the micro-calcification of a position spaced apart from each other as the third focal point f3 to which the third ultrasound pulse is focused.

In the case of FIG. 5, the first position to the third position in which the micro-calcification exists are located at different depths and are located at positions spaced apart from each other in a longitudinal direction. On the other hand, in the case of FIG. 5, the first position to the third position in which the micro-calcification exists are located at different depths, but exist on a straight line in the longitudinal direction.

The controller 120 may obtain second to fourth data by repeating an operation of transmitting the second ultrasound pulse to the first position to the third position of the object through the ultrasound transceiver 110 and receiving the echo signal reflected from the first position to the third position the plurality of times at predetermined time intervals.

In other words, according to the ultrasound diagnostic apparatus 100, in order to detect the micro-calcifications present at different depths and different positions in the object, by setting a plurality of focal points to which second ultrasound pulses are focused, and transmitting the second ultrasound pulses to each of the set focal points, the micro-calcifications tissues present at different depths may be effectively detected.

The controller 120 may control the ultrasound transceiver 110 so that the second ultrasound pulses transmitted to the plurality of positions where the micro-calcification tissues, such as the first position to the third position of the object are present, are transmitted at predetermined time intervals. That is, the controller 120 may control the ultrasound transceiver 110 so that the second ultrasound pulse is transmitted to the first focal point f1 where the micro-calcification tissues are present and the second ultrasound pulse is transmitted to the second focal point f2 after a certain period of time.

In addition, the controller 120 may control the ultrasound transmitter 110 so that the second ultrasound pulses transmitted to the plurality of positions of the object are simultaneously transmitted. That is, by setting an area in which the ultrasound pulses are transmitted in a transducer array provided in the probe 20 in advance, the controller 120 may control the ultrasound transceiver 110 so that the second ultrasound pulses are simultaneously transmitted to the plurality of focal points of the object in each predetermined area of the transducer array.

As described above, in the micro-calcification tissues present in the object, a plurality of tissues may exist, and the existing areas may be distributed at different depths or positions. However, there is no limit the number of positions where the second ultrasound pulse is focused. However, in the following, for convenience of description, it is assumed that the micro-calcification tissues exist in the first and second positions of the object, and the focal points to be set are also the first focal point f1 and the second focal point f2.

The second ultrasound pulse may be a pulse designed based on the characteristics of the micro-calcification tissues. The second ultrasound pulse may be a pulse having a predefined sequence of a waveform size, a phase sign, a period, etc. to detect micro-calcification.

For example, the second ultrasound pulse may be designed such that a negative pressure component is a predominant asymmetric pulse compared to a positive pressure component, and has a longer wavelength than the first ultrasound pulse.

The controller 120 may obtain data including information about the echo signal reflected from the object from the ultrasound transceiver 110. That is, the controller 120 may obtain the first data from the ultrasound transceiver 110. In addition, the controller 120 may obtain the second data from the first position of the object, and may obtain the third data from the second position of the object. The controller 120 may obtain RF channel data or complex baseband I/Q data that has passed through an ADC.

The controller 120 may detect the micro-calcification tissues present at different positions in the object by analyzing the second data and the third data. The controller 120 may reconstruct the second data and the third data into three-dimensional (3D) data including information about an axial depth, a lateral width, and a time axis, and may extract the micro-calcification tissues by analyzing the 3D data. The 3D data may be spatiotemporal data based on the axial depth, the lateral area, and the time axis.

For example, the controller 120 may estimate at least one of an intensity, frequency, and phase of the echo signal reflected from each area of the object by applying singular value decomposition (SVD) to the second data and the third data, based on the estimated value, the micro-calcification tissues in the object may be detected.

The controller 120 may calculate a dispersion of a phase change over time of the echo signal reflected from each area of the object based on the second data and detect an area in which the dispersion of the phase change is greater than or equal to a predetermined value as the micro-calcification tissues in the object.

The display 140 may display the ultrasound image generated based on the first data, and may display the image representing the micro-calcification tissues detected at the first and second positions of the object.

The display 140 may independently display the ultrasound image and the image representing the micro-calcification in different areas, respectively. In addition, the display 140 may display a single image by fusing a B-mode image of a cross section of the object and the image representing the micro-calcification. In addition, the display 140 may display an area corresponding to the detected micro-calcification tissues on the ultrasound image, which is the B-mode image of the cross section of the object.

In particular, the display 140 may independently display the micro-calcification tissues present at different positions within the object.

The display 140 may display a color bar representing a plurality of colors corresponding to values representing characteristics of the micro-calcification tissues, select a color from the color bar based on values representing characteristics of the detected micro-calcification tissues, and display the selected color in the area corresponding to the detected micro-calcification tissues included in the ultrasound image. For example, a value representing the characteristics of the micro-calcification tissues may represent a roughness of the phase change over time of the echo signal reflected from the microscopic tissue.

Referring to FIG. 4, the controller 120 may include a pulse generator 210, a spatiotemporal data obtainer 220, an ultrasound image generator 230, a micro-calcification detector 240, and a micro-calcification image generator 250. Each of the blocks 210, 220, 230, 240, and 250 included in the controller 120 illustrated in FIG. 4 may be an individual hardware configuration or may be functional blocks implemented by the controller 120. Accordingly, the operations of the blocks 210, 220, 230, 240, and 250 described below may be performed by the controller 120.

The pulse generator 210 may generate the ultrasound pulse to be transmitted to the object through the probe 20. The pulse generator 210 may control the ultrasound transceiver 110 to form the transmission signal to be applied to each of the plurality of transducers in consideration of the positions and focal points of the plurality of transducers included in the probe 20. The pulse generator 210 may transmit the ultrasound pulse by controlling the ultrasound transceiver 110.

The pulse generator 210 may generate not only a gray scale ultrasound image of the object scanned according to an amplitude mode (A-mode), the B-mode, and a motion mode (M-mode), but also the ultrasound pulse designed to obtain a Doppler image representing the movement of the object.

The pulse generator 210 may generate a pre-designed first ultrasound pulse to obtain the B-mode image. In addition, the pulse generator 210 may generate a pre-designed second ultrasound pulse for micro-calcification detection. The pulse generator 210 may generate the second ultrasound pulse in which transmission parameters such as a sign, a magnitude, a phase, a period, etc. of a waveform are determined in advance based on the micro-calcification characteristic.

With respect to the second ultrasound pulse transmitted by the ultrasound diagnostic apparatus 100 to detect the micro-calcification, it will be described in detail below with reference to FIGS. 7 to 9.

The pulse generator 210 may generate the ultrasound pulse having a symmetrical (+)/(-) size as the first ultrasound pulse, and may generate a pulse having an asymmetrical (+)/(-) size as the second ultrasound pulse. The pulse generator 210 may improve a micro-calcification detection performance by using an asymmetric ultrasound pulse.

For example, as illustrated in FIG. 7, the asymmetric ultrasound pulse may include a pulse in which a positive pressure is dominant as in a graph 510 or a pulse in which a negative pressure is dominant as in a graph 520. The pulse illustrated in FIG. 7 may be designed in a form in which a harmonic component is added to a transmission center frequency f0 as illustrated in [Equation 1] below. The harmonic component may be designed in a form in which a 2f0 component is multiplied by a weight of a0.

[Equation 1]

$$f_n = f_0 + 2f_0 \cdot a_0$$

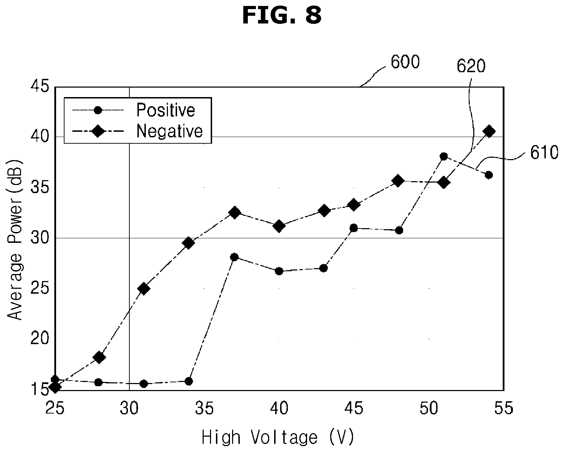

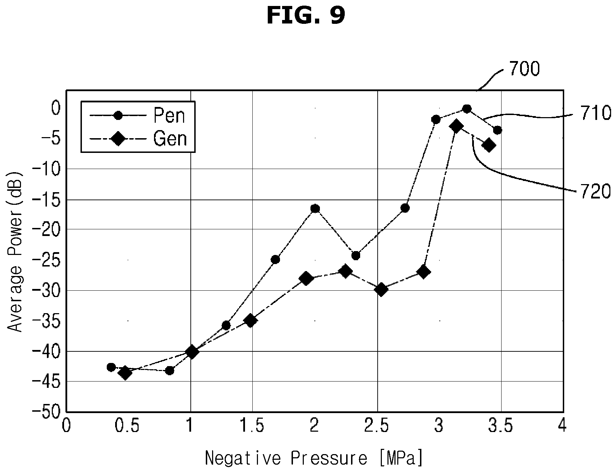

The ultrasound pulse optimized for micro-calcification detection may be designed based on experimental results of FIGS. 8 and 9.

The graph 600 of FIG. 8 illustrates an average value of the micro-calcification signal received from the object when the ultrasound pulse of the waveform illustrated in FIG. 7 is applied to the object including a phantom simulating breast micro-calcification.

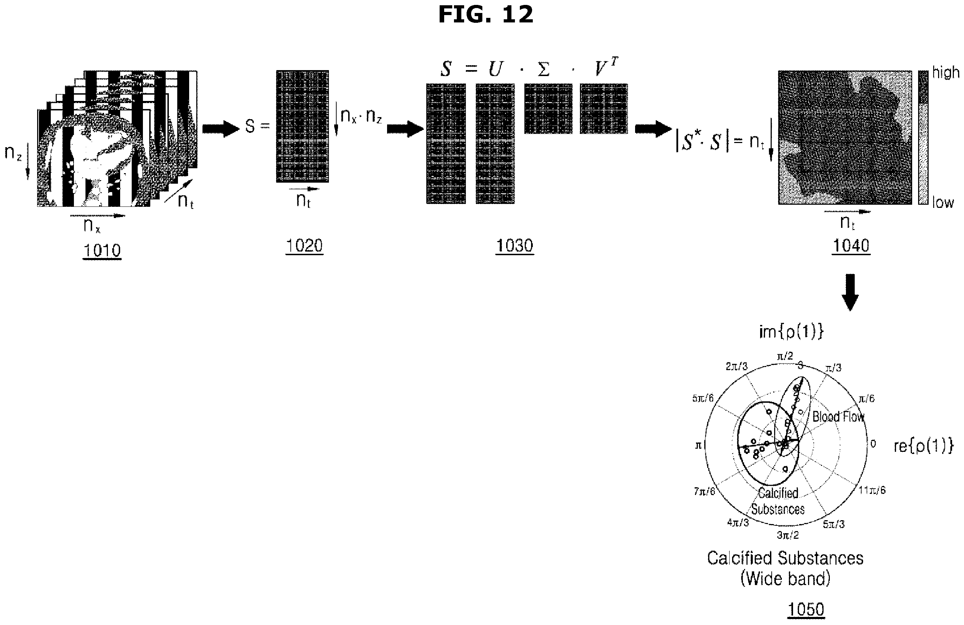

The micro-calcification signal may refer to a signal of a micro-calcification area extracted based on data obtained from the echo signal received from the object. According to an embodiment, the micro-calcification signal may be obtained through processes illustrated in FIGS. 10 and 12, and a specific method of obtaining the micro-calcification signal will be described later.

The graph 610 of FIG. 8 illustrates a change in the average value of the micro-calcification signal received from the object when a high voltage of the ultrasound pulse dominated by the positive pressure illustrated in the graph 510 of FIG. 7 is increased. The graph 620 of FIG. 8 illustrates the change in the average value of the micro-calcification signal received from the object when the high voltage of the ultrasound pulse dominated by the positive pressure illustrated in the graph 520 of FIG. 7 is increased.

According to the experimental results illustrated in FIG. 8, it can be seen that when the ultrasound pulse with a predominant negative pressure is used, the micro-calcification signal is detected larger than when the ultrasound pulse with a predominance positive pressure is used. Therefore, the pulse generator 210 may preferably generate the ultrasound pulse with the predominant negative pressure as the second ultrasound pulse.

The graph 700 of FIG. 9 illustrates the average value of the micro-calcification signal received from the object when the ultrasound pulse of the waveform illustrated in the graph 520 of FIG. 7 is applied to the object including the phantom simulating breast micro-calcification.

The graph 700 of FIG. 9 illustrates the change in the average value of the micro-calcification signal received from the object when the negative pressure illustrated in the graph 520 of FIG. 7 increases the negative pressure of the predominant ultrasound pulse. The graph 710 of FIG. 9 illustrates the change in the average value of the micro-calcification signal received from the object when the negative pressure having a low (penetration) frequency increases the negative pressure of the predominant ultrasound pulse. The graph 720 of FIG. 9 illustrates the change in the average value of the micro-calcification signal received from the object when the negative pressure having a general frequency increases the negative pressure of the predominant ultrasound pulse.

According to the experimental results illustrated in FIG. 9, it can be seen that when the ultrasound pulse having the low frequency (long wavelength) is used, the micro-calcification signal is detected larger than when the ultrasound pulse having a relatively high frequency (short wavelength) is used. Accordingly, the pulse generator 210 may preferably generate the ultrasound pulse having the wavelength longer than the predetermined value as the second ultrasound pulse. In addition, according to the experimental results illustrated in FIG. 9, it can be seen that as the negative pressure of the ultrasound pulse increases, the average value of the micro-calcification signal received from the object increases.

Therefore, the pulse generator 210 may preferably generate the ultrasound pulse having the negative pressure greater than the predetermined value as the second ultrasound pulse. For example, the pulse generator 210 may generate the ultrasound pulse in which the magnitude of the negative pressure is greater than the magnitude of the negative pressure of the first ultrasound pulse as the second ultrasound pulse.

Returning to FIG. 4 again, the structure of the controller 120 of the ultrasound diagnostic apparatus 100 will be described.

The spatiotemporal data obtainer 220 may reconstruct the ultrasound data reflected from the object into spatiotemporal data including spatiotemporal information. The spatiotemporal data obtainer 220 may obtain spatiotemporal data from the second data obtained from the echo signal reflected from the object in response to the second ultrasound pulse transmitted to the first position and the second position of the object.

The ultrasound diagnostic apparatus 100 may obtain the second data by repeatedly performing an operation of transmitting the second ultrasound pulse to the first position in which the micro-calcification of the object exists through the probe 20 and receiving the echo signal at a predefined number of times and time intervals. In addition, the ultrasound diagnostic apparatus 100 may obtain the third data by repeatedly performing an operation of transmitting the second ultrasound pulse to the second position, which is a position different from the first position of the object, and receiving the echo signal at the predefined number of times and time intervals.

The second data and the third data may include a plurality of image data obtained with respect to the cross section of the object at a predetermined period, and each image data may include spatial information about the cross section of the object.

As illustrated in FIG. 10, the spatiotemporal data obtainer 220 may be reconstructed into the 3D data 801 including information about the axial depth, the lateral width, and the time by arranging the plurality of image data included in the second data and the third data on the time axis. Since the data obtained by the spatiotemporal data obtainer 220 includes both temporal information and spatial information, it may be described as spatiotemporal data.

The micro-calcification detector 240 may independently detect the spatiotemporal data obtained from the spatiotemporal data obtainer 220 by distinguishing between general tissues and the micro-calcification through spatiotemporal characteristic analysis. For example, the micro-calcification detector 240 may independently detect the micro-calcification signal by performing singular value decomposition 440 on spatiotemporal data. In addition, the micro-calcification detector 240 may perform independent micro-calcification signal detection based on a power or an average frequency of the micro-calcification signal, a phase change, etc. using an autocorrelation function. For example, the micro-calcification detector 240 may utilize a micro-calcification power estimation value based on the autocorrelation function.

The micro-calcification detector 240 may separate and detect the micro-calcification signal from the spatiotemporal data based on the characteristics of the micro-calcification. For example, the micro-calcification has a random phase change characteristic compared to the general tissues. It is known that this is due to a non-uniform nature of a surface of the calcification tissue.

The graph 910 of FIG. 11 illustrates a phase change of a signal of a blood flow area extracted based on data obtained in response to the transmitted ultrasound pulse when the ultrasound pulse is transmitted to the object including a blood vessel a plurality of times. The graph 920 of FIG. 11 illustrates the phase change of the signal of the micro-calcification area extracted based on data obtained in response to the transmitted ultrasound pulse when the ultrasound pulse is transmitted to the object including the micro-calcification the plurality of times.

As illustrated in FIG. 11, when compared with the general tissues such as the blood flow, it can be seen that the micro-calcification has the random phase change (i.e., the distribution of the phase change is wide). The micro-calcification detector 240 may separate and detect the micro-calcification signal from the spatiotemporal data based on the phase change characteristic of the micro-calcification. The micro-calcification detector 240 may determine that it is the micro-calcification tissues when the roughness of the phase change is greater than or equal to the predetermined value based on the predetermined value.

FIG. 12 is a view illustrating a method of detecting micro-calcification tissues by a micro-calcification detector according to an embodiment.

The micro-calcification detector 240 may generate a Casorati matrix (x*z, t) to arrange the data in a spatiotemporal manner, based on the spatiotemporal data (1010) obtained by the spatiotemporal data obtainer 220 (1020). The micro-calcification detector 240 may perform the singular value decomposition on the generated Casorati matrix (1030). In this case, an U vector may denote data for spatial information, a V vector denote data for temporal information, and a $\Sigma$ vector may denote a ranking of singular values.

The micro-calcification detector 240 may apply a spatiotemporal signal characteristic analysis function to the obtained singular value vector S. The spatiotemporal signal characteristic analysis function may include a codispersion matrix analysis, the autocorrelation function, and the like. For example, the micro-calcification detector 240 may obtain a spatiotemporal codispersion matrix based on the obtained singular value vector S (1040).

The micro-calcification detector 240 may separate and detect an independent micro-calcification signal by analyzing a spatiotemporal signal characteristic based on a result of applying the spatiotemporal signal characteristic analysis function. For example, the micro-calcification detector 240 may detect the signal in the area having a wide distribution of the phase change over time as the micro-calcification signal (1050).

Returning to FIG. 4 again, the structure of the controller 120 of the ultrasound diagnostic apparatus 100 will be described.

The ultrasound image generator 230 may reconstruct an A-mode image, a B-mode image, an M-mode image, a Doppler image, and the like, based on data received in response to the ultrasound pulse applied to the object. The ultrasound image generator 230 may generate the B-mode image representing the cross section of the object by reconstructing the first data received in response to the first ultrasound pulse transmitted to the object. The ultrasound image generator 230 may output the generated ultrasound image to the display 140.

The micro-calcification image generator 250 may generate the micro-calcification image by reconstructing the micro-calcification signal independently detected by the micro-calcification detector 240 into a two-dimensional (2D) image. The micro-calcification image generator 250 may generate the micro-calcification image in which the micro-calcification area included in the cross section of the object to which the ultrasound pulse is transmitted is displayed. The micro-calcification image generator 250 may apply various image post-processing to the generated micro-calcification image. The micro-calcification image generator 250 may output the generated micro-calcification image to the display 140.

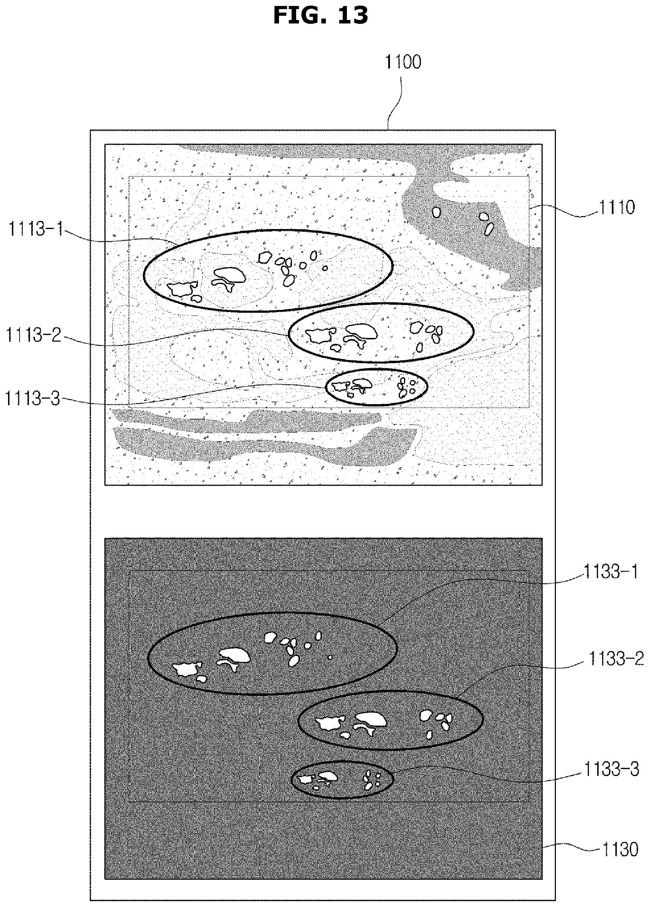

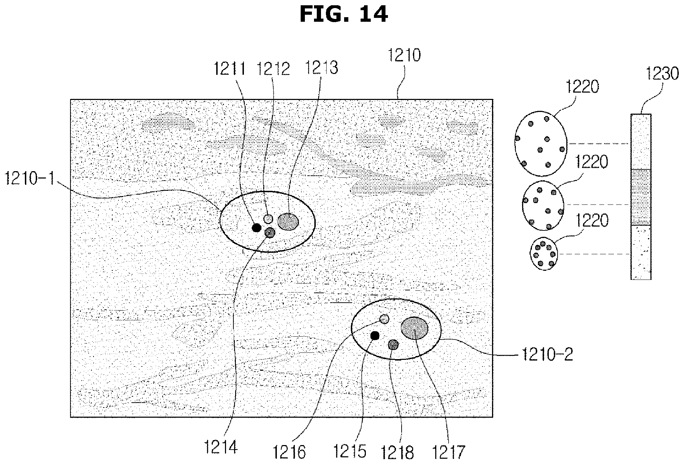

FIGS. 13 and 14 are views illustrating screens displayed on an ultrasound diagnostic apparatus according to an embodiment.

Referring to FIG. 13, the display 140 may independently display an ultrasound image 1110 generated by the controller 120 and an image 1130 representing the micro-calcification in different areas on a screen 1100.

An area 1113 on the ultrasound image 1110 is an area in which the micro-calcification is concentrated in the object, and is the plurality of areas in which the micro-calcification exists, such as an area 1113-1 corresponding to the first position in which the micro-calcification exists to an area 1113-3 corresponding to the third position.

According to the ultrasound diagnostic apparatus 100 according to the embodiment, the image 1130 in which only the micro-calcification area is independently displayed may be generated and displayed. Similar to the ultrasound image 1110, it is identified that micro-calcifications are concentrated in the areas 1133-1, 1113-2, and 1113-3 on the image 1130.

Meanwhile, the embodiment of the disclosure is not limited to the embodiment illustrated in FIG. 13, and a screen in which the ultrasound image and the image representing the micro-calcification are fused may be displayed.

The display 140 may display an image in which the area corresponding to the micro-calcification is displayed on the ultrasound image by fusing the ultrasound image and the image representing the micro-calcification. For example, the area corresponding to the micro-calcification may be displayed as one of a predetermined color, brightness, figure, and symbol on the ultrasound image, or may be displayed to flash.

In addition, the display 140 may simultaneously display the color bar representing the plurality of colors corresponding to values representing characteristics of the micro-calcification tissues. The display 140 may select the color from the color bar based on the value representing characteristics of the detected micro-calcification tissues, and display the selected color in an area corresponding to the detected micro-calcification tissues. However, embodiments are not limited thereto, and various methods for expressing the characteristics of the micro-calcification tissues other than a color bar form may be used.

For example, the value representing the characteristics of micro-calcification tissues may include roughness of the phase of the micro-calcification signal obtained from the corresponding micro-calcification tissues. In general, it is known that the higher the roughness of the phase of the micro-calcification signal, the higher a probability of metastasis to a malignant tumor. Therefore, the ultrasound diagnostic apparatus 100 may increase an accuracy of breast cancer diagnosis by displaying the phase roughness of the micro-calcification signal corresponding to the micro-calcification tissues together with the image representing the micro-calcification tissues.

FIG. 14 illustrates an example of a screen displaying the roughness of the phase as the characteristic of micro-calcification tissues.

The display 140 may display an image 1210 in which areas 1211, 1212, 1213, and 1214 corresponding to the micro-calcifications existing in a first position 1210-1 of the object on the ultrasound image are displayed. For example, the areas 1211, 1212, 1213, and 1214 corresponding to micro-calcification may be displayed in predetermined colors on the ultrasound image.

Likewise, the display 140 may display the image 1210 in which the areas 1215, 1216, 1217, and 1218 corresponding to micro-calcification existing in a second position 1210-2 of the object are displayed on the ultrasound image. For example, the areas 1215, 1216, 1217, and 1218 corresponding to micro-calcification may be displayed in predetermined colors on the ultrasound image.

In addition, the display 140 may also display a color bar 1230 representing the plurality of colors corresponding to values representing characteristics of micro-calcification tissues. An area 1220 of FIG. 14 illustrates that the roughness of the phase of the micro-calcification signal increases toward the top of the color bar 1230. As illustrated in FIG. 14, the display 140 may display the areas 1211 to 1218 in which the micro-calcifications exist by using different colors selected from the color bar, based on the value representing the characteristics of the detected micro-calcification tissues.

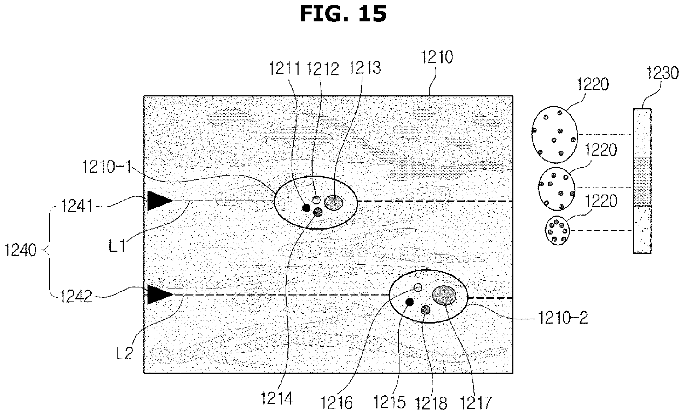

FIG. 15 is a view illustrating an index representing focusing lines in which a plurality of focal points for a second ultrasound pulse are located, according to an embodiment.

Referring to FIG. 15, the display 140 may display an index 1240 indicating focusing lines L1 and L2 in which the first focal point f1 and the second focal point f2 to which the second ultrasound pulse is focused is located in the B-mode image of the cross section of the object.

As described above in FIGS. 5 and 6, the controller 120 may set the first and second positions in which the micro-calcification tissues exist in the object as the first focal point f1 and the second focal point f2 at which the second ultrasound pulse is focused.

The display 140 may display an index 1241 for the focusing line L1 indicating the first focal point f1 set at the first position in which micro-calcification tissues of the object exist. Likewise, the display 140 may display an index 1242 for the focusing line L2 indicating the second focal point f2 set at the second position in which micro-calcification tissues of the object exist.

The user may identify the first position and the second position of the object set as the focal point for micro-calcification detection through a display screen of the display 140. In FIG. 15, a case in which there are two focal points set for micro-calcification detection is described as an example, but when the plurality of micro-calcification tissues exist in the object at different depths or positions, the controller 120 may set two or more focal points to which the second ultrasound pulse is focused, and accordingly, the plurality of focusing lines and indexes 1240 indicating the plurality of focal points may be displayed on the display 140.

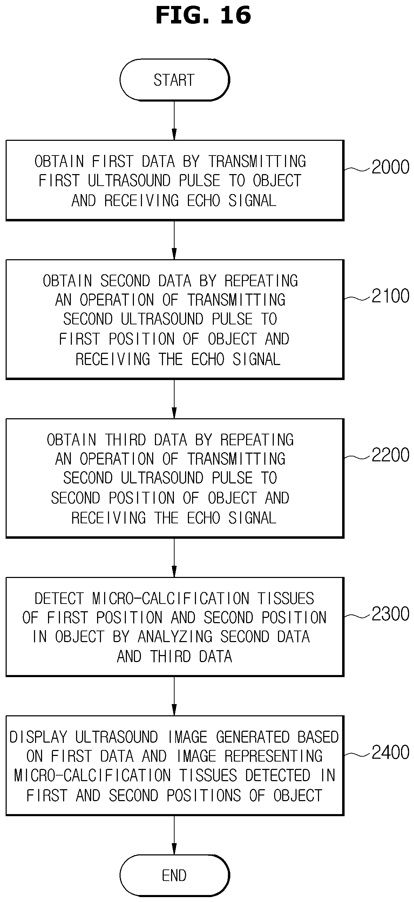

FIG. 16 is a flowchart of a method of displaying an ultrasound image according to an embodiment.

Each operation of a method described below may be performed by each component of the ultrasound diagnostic apparatus 100 illustrated in FIG. 3. The description above with respect to the ultrasound diagnostic apparatus 100 may also be applied to each operation of the following methods.

In operation 2000, the ultrasound diagnostic apparatus 100 may obtain the first data by transmitting the first ultrasound pulse to the object and receiving the echo signal reflected from the object.

In operation 2100, the ultrasound diagnostic apparatus 100 may obtain the second data by repeating the operation of transmitting the second ultrasound pulse different from the first ultrasound pulse to the first position of the object and receiving the echo signal reflected from the object the plurality of times at predetermined time intervals.

Similarly, in operation 2200, the ultrasound diagnostic apparatus 100 may obtain the second data by repeating the operation of transmitting the second ultrasound pulse different from the first ultrasound pulse to the second position of the object and receiving the echo signal reflected from the object the plurality of times at predetermined time intervals.

The second ultrasound pulse may be the pulse designed based on the characteristics of micro-calcification tissues. For example, the second ultrasound pulse is an asymmetric pulse in which the negative pressure component is dominant compared to the positive pressure component, and may have the longer wavelength than the first ultrasound pulse.

In the case of operations 2100 and 2200, when the micro-calcification tissues existing in the object are distributed at different depths at the plurality of positions, the operation may increase in correspondence with the depth at which the micro-calcification tissues exist.

In operation 2300, the ultrasound diagnostic apparatus 100 may detect micro-calcification tissues of the first position and the second position in the object by analyzing the second data and the third data.

The ultrasound diagnostic apparatus 100 may detect only the micro-calcification signal independently from the general tissue by analyzing the received RF signal or the I/Q signal in spatiotemporal manner in response to the ultrasound pulse transmitted to the object. As a method of detecting the micro-calcification signal, a method of estimating power using the spatiotemporal signal characteristic analysis function (e.g., autocorrelation) and estimating the average frequency or dispersion may be used.

Particularly, the ultrasound diagnostic apparatus 100 may reconstruct the second and third data obtained in operations 2100 and 2200 into 3D data including information about the axial depth, the lateral width, and the time. The ultrasound diagnostic apparatus 100 may extract micro-calcification tissues present at different positions within the object by analyzing the 3D data.

The ultrasound diagnostic apparatus 100 may estimate at least one of the intensity, frequency, and phase of the echo signal reflected from each area of the object by applying SVD to the second data and the third data, and may detect the micro-calcification tissues in the object based on the estimated value.

The ultrasound diagnostic apparatus 100 may calculate the dispersion of the phase change over time of the echo signal reflected from each area of the object based on the second data and the third data, and detect the area in which the dispersion of the phase change is more than the predetermined value as the micro-calcification tissues in the object.

In operation 2400, the ultrasound diagnostic apparatus 100 may display the ultrasound image generated based on the first data, and may display the image representing micro-calcification tissues detected in the first and second positions of the object.

The ultrasound diagnostic apparatus 100 may generate the ultrasound image by reconstructing the first data, and may generate the micro-calcification image by reconstructing the micro-calcification signal detected in operation 2300 for a purpose of displaying on the screen.

The ultrasound diagnostic apparatus 100 may individually display the ultrasound images and the images representing micro-calcification tissues in different areas. Alternatively, the ultrasound diagnostic apparatus 100 may display a single image by fusing the ultrasound image and the image representing micro-calcification tissues. The ultrasound diagnostic apparatus 100 may display the single image, indicating the area corresponding to micro-calcification tissues detected on the ultrasound image.

For example, the ultrasound image generated based on the first data is the B-mode image representing the cross section of the object, and the image representing micro-calcification tissues may be the image displaying the position of micro-calcification tissues within the cross section by color, contrast, symbols, figures, blinking, and the like.

The ultrasound diagnostic apparatus 100 may further display the color bar representing the plurality of colors corresponding to values representing characteristics of micro-calcification tissues. The ultrasound diagnostic apparatus 100 may select the color from the color bar based on the value representing the characteristic of the detected micro-calcification tissues, and display the selected color in the area corresponding to the detected micro-calcification tissues. For example, the value representing characteristics of micro-calcification tissues may be the value representing roughness of the phase change over time of the echo signal reflected from the micro-calcification tissues.

As described above, according to the embodiments of the disclosure, the ultrasound diagnostic apparatus may accurately detect micro-calcification tissues distributed at different depths or positions, and may independently display only micro-calcification images, or display images representing micro-calcification by fusion with ultrasound B-mode images. In addition, by accurately detecting micro-calcification, not only can breast cancer be diagnosed early, but also micro-calcification generated in organs other than breasts as calcium is accumulated in the human body can be monitored, thereby improving the accuracy of diagnosis of various diseases.

The disclosed embodiments may be implemented as S/W program including instructions stored in a computer-readable storage medium.

The computer may include the ultrasound diagnostic apparatus according to the disclosed embodiments as a device capable of calling a stored command from the storage medium and operating according to the disclosed embodiments according to the called command.

The computer-readable storage medium may be provided in the form of a non-transitory storage medium. Here, 'non-transitory' may refer to that the storage medium does not contain a signal and is tangible, but does not distinguish between semi-permanent or temporary storage of data in the storage medium.

In addition, the ultrasound diagnostic apparatus or method according to the disclosed embodiments may be provided by being included in a computer program product. The computer program product is commodities that can be traded between sellers and buyers.

The computer program product may include the S/W program and the storage medium readable by the computer in which the S/W program is stored. For example, the computer program product includes a manufacturer of the ultrasound diagnostic apparatus or a product (e.g., a downloadable app) in the form of the S/W program that is electronically distributed through an electronic market (e.g., Google Play Store, App Store). For electronic distribution, at least a part of the S/W program may be stored in the storage medium or may be temporarily generated. In this case, the storage medium may be a server of the manufacturer, the server of the electronic market, or the storage medium of a relay server temporarily storing SW programs.

The computer program product may include the storage medium of the server or the storage medium of a terminal in a system composed of the server and a terminal (e.g., ultrasound diagnostic apparatus). Alternatively, when there is a third device (e.g., a smartphone) that is communicatively connected to the server or the terminal, the computer program product may include the storage medium of the third device. Alternatively, the computer program product may include the S/W program itself transmitted from the server to the terminal or the third device, or transmitted from the third device to the terminal.

In this case, one of the server, the terminal, and the third device may execute the computer program product to perform the method according to the disclosed embodiments. Alternatively, two or more of the server, the terminal, and the third device may execute the computer program product to distribute the method according to the disclosed embodiments.

For example, a server (e.g., a cloud server or an artificial intelligence server) may execute the computer program product stored in the server, and control the terminal connected to the server to perform the method according to the disclosed embodiments.

As another example, by executing the computer program product, the third device may control the terminal connected in communication with the third device to perform the method according to the disclosed embodiment. As a specific example, the third device may control the ultrasound diagnostic apparatus to remotely control the ultrasound diagnostic apparatus to irradiate the ultrasound signal as the object and to generate the image of the portion inside the object based on signal information reflected from the object.

As another example, the third device may execute the computer program product to directly perform the method according to the disclosed embodiment based on a value input from an auxiliary device (e.g., the probe of the medical apparatus). As a specific example, the auxiliary device may irradiate the ultrasound signal as the object and obtain the ultrasound signal reflected from the object. The third device may receive reflected signal information from the auxiliary device, and may generate the image of the portion inside the object based on the input signal information.

When the third device executes the computer program product, the third device may download the computer program product from the server and execute the downloaded computer program product. Alternatively, the third device may perform the method according to the disclosed embodiments by executing the computer program product provided in a preloaded state.

Embodiments of the disclosure have been described with reference to the accompanying drawings. It should be obvious to a person of ordinary skill in the art that the disclosure may be practiced in other forms by modifying and substituting some or all of the components of the embodiments as described above without changing the technical idea or essential features of the disclosure. The above embodiments are only by way of example, and should not be interpreted as a limitation to the embodiments.

**Claims**

1. An ultrasound diagnostic apparatus (100) comprising:

   a probe (20);
   an ultrasound transceiver (110) configured to transmit an ultrasound signal to an object through the probe and receive an echo signal reflected from the object;
   a controller (120) configured to:

   obtain first data by controlling the ultrasound transceiver (110) to transmit a first ultrasound pulse to the object and receive the echo signal reflected from the object,
   obtain second data by controlling the ultrasound transceiver (110) to repeat an operation of transmitting a second ultrasound pulse different from the first ultrasound pulse to a first position of the object and receiving the echo signal reflected from the first position of the object a plurality of times at predetermined time intervals,
   obtain third data by controlling the ultrasound transceiver (110) to repeat an operation of transmitting the second ultrasound pulse to a second position of the object and receiving the echo signal reflected from the second position of the object the plurality of times at predetermined time intervals, and detect the micro-calcification tissues of the first position and the second position by analyzing the obtained second and third data; and

   a display (140) configured to display an ultrasound image generated based on the obtained first data and an image representing micro-calcification tissues detected in the first position and the second position,
   **characterized in, that** the display (140) is configured to:

   display a color bar representing a plurality of colors corresponding to a dispersion of a phase change over time of the echo signal reflected from the micro-calcification tissues;
   select a color from the color bar based on the dispersion of the phase change of the echo signal reflected from the micro-calcification tissues; and
   display the selected color in an area corresponding to the detected micro-calcification tissues included in the ultrasound image.

2. The ultrasound diagnostic apparatus according to claim 1, wherein:

   the first position of the object is a position that is a different depth from the second position and is spaced apart from each other; and
   the controller is configured to set the first position and the second position of the object as a first focal point and a second focal point to which the transmitted second ultrasound pulse is focused, respectively.

3. The ultrasound diagnostic apparatus according to claim 1, wherein the controller is configured to control the ultrasound transceiver so that the second ultrasound pulse transmitted to the first position of the object and the second ultrasound pulse transmitted to the second position of the object are transmitted at predetermined time intervals.

4. The ultrasound diagnostic apparatus according to claim 1, wherein the controller is configured to control the ultrasound transceiver so that the second ultrasound pulse transmitted to the first position of the object and the second ultrasound pulse transmitted to the second position of the object are simultaneously transmitted.

5. The ultrasound diagnostic apparatus according to claim 1, wherein the second ultrasound pulse is an asymmetric pulse in which a negative pressure component is dominant compared to a positive pressure component, and has a longer wavelength than the first ultrasound pulse.

6. The ultrasound diagnostic apparatus according to claim 1, wherein, in detecting the micro-calcification tissues, the controller is configured to reconstruct the obtained second and third data into three-dimensional (3D) data including information about an axial depth, a lateral width, and a time by arranging a plurality of image data included in the second data and the third data on the time axis, and to extract the micro-calcification tissues by analyzing the 3D data.

7. The ultrasound diagnostic apparatus according to claim 1, wherein, in detecting the micro-calcification tissues, the controller is configured to estimate at least one of an intensity, frequency, or phase of the echo signal reflected from each area of the object by applying singular value decomposition (SVD) to the obtained second and third data, and to detect the micro-calcification tissues in the object based on the estimated value.

8. The ultrasound diagnostic apparatus according to claim 1, wherein, in detecting the micro-calcification tissues, the controller is configured to calculate a dispersion of a phase change over time of the echo signal reflected from each area of the object based on the obtained second and third data, and to detect an area in which the dispersion of the phase change is greater than or equal to a predetermined value as the micro-calcification tissues in the object.

9. The ultrasound diagnostic apparatus according to claim 1, wherein the display is configured to display an index indicating focusing lines in which the set first focal point and second focal point are located in a brightness mode (B-mode) image of a cross section of the object.

10. The ultrasound diagnostic apparatus according to claim 1, wherein:

the ultrasound image is a brightness mode (B-mode) image for a cross section of the object; and
the display is configured to display an area corresponding to the detected micro-calcification tissues on the ultrasound image.

11. A method of controlling an ultrasound diagnostic apparatus comprising:

obtaining, by a controller, first data by transmitting a first ultrasound pulse to the object and receiving the echo signal reflected from the object;

obtaining, by the controller, second data by repeating an operation of transmitting a second ultrasound pulse different from the first ultrasound pulse to a first position of the object and receiving the echo signal reflected from the first position of the object a plurality of times at predetermined time intervals;
obtaining, by the controller, third data by repeating an operation of transmitting the second ultrasound pulse to a second position of the object and receiving the echo signal reflected from the second position of the object the plurality of times at predetermined time intervals;
detecting, by the controller, the micro-calcification tissues of the first position and the second position by analyzing the obtained second and third data; and
displaying, by a display, an ultrasound image generated based on the obtained first data and an image representing micro-calcification tissues detected in the first position and the second position,
wherein the displaying the image representing micro-calcification tissues includes:

displaying a color bar representing a plurality of colors corresponding to a dispersion of a phase change over time of the echo signal reflected from the micro-calcification tissues;
selecting a color from the color bar based on the dispersion of the phase change of the echo signal reflected from the micro-calcification tissues; and
displaying the selected color in an area corresponding to the detected micro-calcification tissues included in the ultrasound image.

12. The method according to claim 11, wherein the first position of the object is a position that is a different depth from the second position and is spaced apart from each other, and
the method further comprises:
setting, by the controller, the first position and the second position of the object as a first focal point and a second focal point to which the transmitted second ultrasound pulse is focused, respectively.

**Patentansprüche**

1. Ultraschalldiagnosevorrichtung (100), die Folgendes umfasst:

eine Sonde (20);
einen Ultraschall-Transceiver (110), der dazu konfiguriert ist, durch die Sonde ein Ultraschallsignal an ein Objekt zu senden und ein von dem Objekt reflektiertes Echosignal zu empfangen; eine Steuerung (120), die zu Folgendem konfiguriert ist:

Erhalten erster Daten durch Steuern des Ultraschall-Transceivers (110), um einen ersten Ultraschallimpuls an das Objekt zu senden und das von dem Objekt reflektierte Echosignal zu empfangen,

Erhalten zweiter Daten durch Steuern des Ultraschall-Transceivers (110), um einen Vorgang des Sendens eines zweiten Ultraschallimpulses, der sich von dem ersten Ultraschallimpuls unterscheidet, an eine erste Position des Objekts und des Empfangens des von der ersten Position des Objekts reflektierten Echosignals mehrere Male in vorgegebenen Zeitintervallen zu wiederholen,

Erhalten dritter Daten durch Steuern des Ultraschall-Transceivers (110), um einen Vorgang des Sendens des zweiten Ultraschallimpulses an eine zweite Position des Objekts und des Empfangens des von der zweiten Position des Objekts reflektierten Echosignals die mehreren Male in vorgegebenen Zeitintervallen zu wiederholen, und

Erkennen der Mikroverkalkungsgewebe der ersten Position und der zweiten Position durch Analysieren der erhaltenen zweiten und dritten Daten; und

eine Anzeige (140), die so konfiguriert ist, dass sie ein basierend auf den erhaltenen ersten Daten erzeugtes Ultraschallbild und ein Bild anzeigt, das in der ersten Position und der zweiten Position erkannte Mikroverkalkungsgewebe darstellt,

**dadurch gekennzeichnet, dass** die Anzeige (140) zu Folgendem konfiguriert ist:

Anzeigen eines Farbbalkens, der eine Vielzahl von Farben darstellt, die einer zeitlichen Streuung einer Phasenänderung des Echosignals, das von den Mikroverkalkungsgeweben reflektiert wird, entsprechen;

Auswählen einer Farbe aus dem Farbbalken basierend auf der Streuung der Phasenänderung des Echosignals, das von den Mikroverkalkungsgeweben reflektiert wird; und

Anzeigen der ausgewählten Farbe in einem Bereich, der den in dem Ultraschallbild enthaltenen erkannten Mikroverkalkungsgeweben entspricht.

2. Ultraschalldiagnosevorrichtung nach Anspruch 1, wobei:

es sich bei der ersten Position des Objekts um eine Position handelt, die von der zweiten Position beabstandet ist und eine andere Tiefe als die zweite Position aufweist; und

die Steuerung so konfiguriert ist, dass sie die erste Position und die zweite Position des Objekts als einen ersten Fokuspunkt bzw. einen zweiten Fokuspunkt einstellt, auf die der gesendete zweite Ultraschallimpuls fokussiert wird.

3. Ultraschalldiagnosevorrichtung nach Anspruch 1, wobei die Steuerung dazu konfiguriert ist, den Ultraschall-Transceiver so zu steuern, dass der an die erste Position des Objekts gesendete zweite Ultraschallimpuls und der an die zweite Position des Objekts gesendete zweite Ultraschallimpuls in vorgegebenen Zeitintervallen gesendet werden.

4. Ultraschalldiagnosevorrichtung nach Anspruch 1, wobei die Steuerung dazu konfiguriert ist, den Ultraschall-Transceiver so zu steuern, dass der an die erste Position des Objekts gesendete zweite Ultraschallimpuls und der an die zweite Position des Objekts gesendete zweite Ultraschallimpuls gleichzeitig gesendet werden.

5. Ultraschalldiagnosevorrichtung nach Anspruch 1, wobei es sich bei dem zweiten Ultraschallimpuls um einen asymmetrischen Impuls handelt, bei dem eine Unterdruckkomponente im Vergleich zu einer Überdruckkomponente dominiert, und der eine längere Wellenlänge als der erste Ultraschallimpuls aufweist.

6. Ultraschalldiagnosevorrichtung nach Anspruch 1, wobei die Steuerung beim Erkennen der Mikroverkalkungsgewebe zu Folgendem konfiguriert ist: Rekonstruieren der erhaltenen zweiten und dritten Daten in dreidimensionale (3D) Daten, einschließlich Informationen über eine axiale Tiefe, eine seitliche Breite und eine Zeit, indem eine Vielzahl von Bilddaten, die in den zweiten Daten und den dritten Daten enthalten sind, auf der Zeitachse angeordnet wird, und Extrahieren des Mikroverkalkungsgewebes durch Analyse der 3D-Daten.

7. Ultraschalldiagnosevorrichtung nach Anspruch 1, wobei die Steuerung beim Erkennen der Mikroverkalkungsgewebe zu Folgendem konfiguriert ist: Schätzen einer Intensität, Frequenz und/oder Phase des Echosignals, das von jedem Bereich des Objekts reflektiert wird, indem eine Singulärwertzerlegung (SWZ) auf die erhaltenen zweiten und dritten Daten angewendet wird, und Erkennen des Mikroverkalkungsgewebes in dem Objekt basierend auf dem geschätzten Wert.

8. Ultraschalldiagnosevorrichtung nach Anspruch 1, wobei die Steuerung beim Erkennen der Mikroverkalkungsgewebe zu Folgendem konfiguriert ist: Berechnen einer zeitlichen Streuung einer Phasenänderung des Echosignals, das von jedem Bereich des Objekts reflektiert wird, basierend auf den erhaltenen zweiten und dritten Daten, und Erkennen eines Bereichs, in dem die Streuung der Phasenänderung größer oder gleich einem vorbestimmten Wert ist, als das Mikroverkalkungsgewebe in dem Objekt.

9. Ultraschalldiagnosevorrichtung nach Anspruch 1, wobei die Anzeige so konfiguriert ist, dass sie einen Index anzeigt, der Fokussierungslinien angibt, in denen sich der eingestellte erste Fokuspunkt und der eingestellte zweite Fokuspunkt in einem Bild im Helligkeitsmodus (B-Modus) eines Querschnitts des Objekts befinden.

10. Ultraschalldiagnosevorrichtung nach Anspruch 1, wobei:

es sich bei dem Ultraschallbild um ein Bild im Helligkeitsmodus (B-Modus) für einen Querschnitt des Objekts handelt; und
die Anzeige so konfiguriert ist, dass sie auf dem Ultraschallbild einen Bereich anzeigt, der den erkannten Mikroverkalkungsgeweben entspricht.

11. Verfahren zur Steuerung einer Ultraschalldiagnosevorrichtung, wobei das Verfahren Folgendes umfasst:

durch eine Steuerung, Erhalten erster Daten durch Senden eines ersten Ultraschallimpulses an das Objekt und Empfangen des von dem Objekt reflektierten Echosignals;
durch die Steuerung, Erhalten zweiter Daten durch Wiederholen eines Vorgangs des Sendens eines zweiten Ultraschallimpulses, der sich von dem ersten Ultraschallimpuls unterscheidet, an eine erste Position des Objekts, und des Empfangens des von der ersten Position des Objekts reflektierten Echosignals mehrere Male in vorgegebenen Zeitintervallen;
durch die Steuerung, Erhalten dritter Daten durch Wiederholen eines Vorgangs des Sendens des zweiten Ultraschallimpulses an eine zweite Position des Objekts und des Empfangens des von der zweiten Position des Objekts reflektierten Echosignals die mehreren Male in vorgegebenen Zeitintervallen;
durch die Steuerung, Erkennen der Mikroverkalkungsgewebe der ersten Position und der zweiten Position durch Analysieren der erhaltenen zweiten und dritten Daten; und
durch eine Anzeige, Anzeigen eines basierend auf den erhaltenen ersten Daten erzeugten Ultraschallbildes und eines Bildes, das in der ersten Position und der zweiten Position erkannte Mikroverkalkungsgewebe darstellt,
wobei das Anzeigen des die Mikroverkalkungsgewebe darstelltenden Bildes Folgendes umfasst:

Anzeigen eines Farbbalkens, der eine Vielzahl von Farben darstellt, die einer zeitlichen Streuung einer Phasenänderung des Echosignals, das von den Mikroverkalkungsgeweben reflektiert wird, entsprechen;
Auswählen einer Farbe aus dem Farbbalken basierend auf der Streuung der Phasenänderung des Echosignals, das von den Mikroverkalkungsgeweben reflektiert wird; und
Anzeigen der ausgewählten Farbe in einem Bereich, der den in dem Ultraschallbild enthaltenen erkannten Mikroverkalkungsgeweben entspricht.

12. Verfahren nach Anspruch 11, wobei es sich bei der ersten Position des Objekts um eine Position handelt, die von der zweiten Position beabstandet ist und eine andere Tiefe als die zweite Position aufweist, und
das Verfahren ferner Folgendes umfasst:
durch die Steuerung, Einstellen der ersten Position und der zweiten Position des Objekts als einen ersten Fokuspunkt bzw. einen zweiten Fokuspunkt, auf die der gesendete zweite Ultraschallimpuls fokussiert wird.

**Revendications**

1. Appareil d'échographie diagnostique (100) comprenant :

une sonde (20),
un transducteur ultrasonore (110) conçu pour émettre un signal ultrasonore en direction d'un objet à travers la sonde et pour recevoir un signal d'écho réfléchi par l'objet,
un organe de commande (120) conçu pour :

obtenir des premières données en commandant l'émission, par le transducteur ultrasonore (110), d'une première impulsion ultrasonore en direction de l'objet, et la réception du signal d'écho réfléchi par l'objet,
obtenir des deuxièmes données en commandant la réitération, une pluralité de fois à intervalles de temps prédéterminés, d'une opération d'émission, par le transducteur ultrasonore (110), d'une deuxième impulsion ultrasonore différente de la première impulsion ultrasonore en direction d'un premier point de l'objet, et de réception du signal d'écho réfléchi par le premier point de l'objet,

obtenir des troisièmes données en commandant la réitération, une même pluralité de fois à intervalles de temps prédéterminés, d'une opération d'émission, par le transducteur ultrasonore (110), de la deuxième impulsion ultrasonore en direction d'un deuxième point de l'objet et de réception du signal d'écho réfléchi par le deuxième point de l'objet, et

détecter les tissus microcalcifiés du premier point et du deuxième point en analysant les deuxièmes et troisièmes données obtenues ; et

un écran (140) conçu pour afficher une image échographique générée compte tenu des premières données obtenues et une image représentant les tissus microcalcifiés détectés aux premier et deuxième points ;
**caractérisé en ce que** l'écran (140) est conçu pour :

afficher une barre de couleurs représentant une pluralité de couleurs correspondant à la dispersion dans le temps d'un changement de phase du signal d'écho réfléchi par les tissus microcalcifiés,

sélectionner une couleur dans la barre de couleurs compte tenu de la dispersion du changement de phase du signal d'écho réfléchi par les tissus microcalcifiés, et

afficher la couleur sélectionnée dans une zone correspondant aux tissus microcalcifiés détectés présents dans l'image échographique.

2. Appareil d'échographie diagnostique selon la revendication 1, dans lequel :

le premier point de l'objet est un point qui est à une profondeur différente de celle du deuxième point et qui est espacé de ce dernier, et

l'organe de commande est conçu pour établir le premier point et le deuxième point de l'objet respectivement en tant que premier foyer et deuxième foyer vers lesquels la deuxième impulsion ultrasonore émise est focalisée.

3. Appareil d'échographie diagnostique selon la revendication 1, dans lequel l'organe de commande est conçu pour commander le transducteur ultrasonore de façon que la deuxième impulsion ultrasonore émise en direction du premier point de l'objet et la deuxième impulsion ultrasonore émise en direction du deuxième point de l'objet soient émises à intervalles de temps prédéterminés.

4. Appareil d'échographie diagnostique selon la revendication 1, dans lequel l'organe de commande est conçu pour commander le transducteur ultrasonore de façon que la deuxième impulsion ultrasonore émise en direction du premier point de l'objet et la deuxième impulsion ultrasonore émise en direction du deuxième point de l'objet soient émises simultanément.

5. Appareil d'échographie diagnostique selon la revendication 1, dans lequel la deuxième impulsion ultrasonore est une impulsion asymétrique dans laquelle une composante de pression négative est dominante comparativement à une composante de pression positive pression, et présente une longueur d'onde supérieure à celle de la première impulsion ultrasonore.

6. Appareil d'échographie diagnostique selon la revendication 1, dans lequel, lors de la détection des tissus microcalcifiés, l'organe de commande est conçu pour reconstruire les deuxièmes et troisièmes données obtenues en des données tridimensionnelles (3D) comportant des informations concernant une profondeur axiale, une largeur latérale et un temps, en agençant une pluralité de données d'images présentes dans les deuxièmes données et les troisièmes données sur l'axe temporel, et pour extraire les tissus microcalcifiés par analyse des données 3D.

7. Appareil d'échographie diagnostique selon la revendication 1, dans lequel, lors de la détection des tissus microcalcifiés, l'organe de commande est conçu pour estimer l'intensité, la fréquence et/ou la phase du signal d'écho réfléchi par chaque zone de l'objet, par application d'une décomposition en valeurs singulières (DVS) aux deuxièmes et troisièmes données, et pour détecter les tissus microcalcifiés dans l'objet compte tenu de la valeur estimée.

8. Appareil d'échographie diagnostique selon la revendication 1, dans lequel, lors de la détection des tissus microcalcifiés, l'organe de commande est conçu pour calculer une dispersion dans le temps d'un changement de phase du signal d'écho réfléchi par chaque zone de l'objet, compte tenu des deuxièmes et troisièmes données, et pour détecter une zone dans laquelle la dispersion du changement de phase est supérieure ou égale à une valeur prédéterminée en tant que tissus microcalcifiés dans l'objet.

**9.** Appareil d'échographie diagnostique selon la revendication 1, dans lequel l'écran est conçu pour afficher un indice indiquant des lignes de focalisation dans lesquelles le premier foyer et le deuxième foyer établis sont situés dans une image en mode B d'une section transversale de l'objet.

**10.** Appareil d'échographie diagnostique selon la revendication 1, dans lequel :

l'image échographique est une image en mode B relative à une section transversale de l'objet, et
l'écran est conçu pour afficher, sur l'image échographique, une zone correspondant aux tissus microcalcifiés détectés.

**11.** Procédé de commande d'un appareil d'échographie diagnostique, comprenant :

l'obtention, par un organe de commande, de premières données par émission d'une première impulsion ultrasonore en direction de l'objet et réception du signal d'écho réfléchi par l'objet, l'obtention, par l'organe de commande, de deuxièmes données par réitération, une pluralité de fois à intervalles de temps prédéterminés, d'une opération d'émission d'une deuxième impulsion ultrasonore différente de la première impulsion ultrasonore en direction d'un premier point de l'objet et de réception du signal d'écho réfléchi par le premier point de l'objet, l'obtention, par l'organe de commande, de troisièmes données par réitération, une même pluralité de fois à intervalles de temps prédéterminés, d'une opération d'émission d'une deuxième impulsion ultrasonore en direction d'un deuxième point de l'objet et de réception du signal d'écho réfléchi par le deuxième point de l'objet,
la détection, par l'organe de commande, des tissus microcalcifiés du premier point et du deuxième point par analyse des deuxièmes et troisièmes données obtenues, et
l'affichage, par un écran, d'une image échographique générée compte tenu des premières données obtenues et d'une image représentant les tissus microcalcifiés détectés aux premier et deuxième points ;
ledit affichage de l'image représentant les tissus microcalcifiés comprenant :

l'affichage d'une barre de couleurs représentant une pluralité de couleurs correspondant à la dispersion dans le temps d'un changement de phase du signal d'écho réfléchi par les tissus microcalcifiés ;
la sélection d'une couleur dans la barre de couleurs compte tenu de la dispersion du changement de phase du signal d'écho réfléchi par les tissus microcalcifiés ; et
l'affichage de la couleur sélectionnée dans une zone correspondant aux tissus microcalcifiés détectés présents dans l'image échographique.

**12.** Procédé selon la revendication 11, dans lequel le premier point de l'objet est un point qui est à une profondeur différente de celle du deuxième point et qui est espacé de ce dernier, et
le procédé comprenant en outre :
l'établissement, par l'organe de commande, du premier point et du deuxième point de l'objet respectivement en tant que premier foyer et deuxième foyer vers lesquels la deuxième impulsion ultrasonore émise est focalisée.

# FIG. 1

FIG. 2

(a)

122

121

100a

(b)

171

172

165

122

121

100b

(c)

20

145

40

100c

# FIG. 3

PROBE 20 ← → ULTRASOUND TRANSCEIVER 110 ← → CONTROLLER 120 ← → DISPLAY 140 · 100

# FIG. 4

```
                                    120
 ┌──────────────────────────────────────────────────────────────────────┐
 │                          CONTROLLER                                    │
 │                                                                        │
 │          210                   240                                     │
 │     ┌──────────────┐    ┌──────────────────┐                           │
 │ ┌──▶│PULSE GENERATOR│    │ MICRO-CALCIFICATION│                        │
 │ │   └──────────────┘    │     DETECTOR      │                          │
 │ │          220          │        241        │        250               │
 │ │   ┌──────────────┐    │  ┌─────────────┐  │   ┌──────────────────┐    │
 │◀┼─▶│ SPATIOTEMPORAL │◀─▶│  │SINGULAR VALUE│  │◀─▶│ MICRO-CALCIFICATION│◀─▶│
 │ │  │ DATA OBTAINER │    │  │DECOMPOSITION│  │   │  IMAGE GENERATOR  │    │
 │ │  └──────────────┘    │  └─────────────┘  │   └──────────────────┘    │
 │ │          230          └──────────────────┘              │            │
 │ │   ┌──────────────┐                                      │            │
 │ └──▶│   ULTRASOUND  │◀─────────────────────────────────────┘            │
 │     │IMAGE GENERATOR│                                                   │
 │     └──────────────┘                                                   │
 └──────────────────────────────────────────────────────────────────────┘
```

# FIG. 5

# FIG. 6

## FIG. 7

Positive pressure dominant pulse

510

Time [us]

Amplitude [V]

Negative pressure dominant pulse

520

Time [us]

Amplitude [V]

## FIG. 8

# FIG. 9

# FIG. 10

# FIG. 11

910

$\text{im}\{\rho(1)\}$

911

Blood Flow
(Narrow band)

920

$\text{im}\{\rho(1)\}$

921

Calcified Substances
(Wide band)

**FIG. 12**

# FIG. 13

# FIG. 14

# FIG. 15

# FIG. 16

START

OBTAIN FIRST DATA BY TRANSMITTING FIRST ULTRASOUND PULSE TO OBJECT AND RECEIVING ECHO SIGNAL —2000

OBTAIN SECOND DATA BY REPEATING AN OPERATION OF TRANSMITTING SECOND ULTRASOUND PULSE TO FIRST POSITION OF OBJECT AND RECEIVING THE ECHO SIGNAL —2100

OBTAIN THIRD DATA BY REPEATING AN OPERATION OF TRANSMITTING SECOND ULTRASOUND PULSE TO SECOND POSITION OF OBJECT AND RECEIVING THE ECHO SIGNAL —2200

DETECT MICRO-CALCIFICATION TISSUES OF FIRST POSITION AND SECOND POSITION IN OBJECT BY ANALYZING SECOND DATA AND THIRD DATA —2300

DISPLAY ULTRASOUND IMAGE GENERATED BASED ON FIRST DATA AND IMAGE REPRESENTING MICRO-CALCIFICATION TISSUES DETECTED IN FIRST AND SECOND POSITIONS OF OBJECT —2400

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012232390 A1